(19) Europäisches Patentamt
European Patent Office
Office européen des brevets

(11) EP 0 409 896 B2

(12) NOUVEAU FASCICULE DE BREVET EUROPEEN

(45) Date de publication et mention de la décision concernant l'opposition:
**05.04.2000 Bulletin 2000/14**

(45) Mention de la délivrance du brevet:
**08.12.1993 Bulletin 1993/49**

(21) Numéro de dépôt: **89905122.1**

(22) Date de dépôt: **19.04.1989**

(51) Int. Cl.[7]: **C12M 1/40**

(86) Numéro de dépôt international:
**PCT/FR89/00182**

(87) Numéro de publication internationale:
**WO 89/10395 (02.11.1989 Gazette 1989/26)**

# (54) ELECTRODE ENZYMATIQUE ET SON PROCEDE DE PREPARATION

ENZYMELEKTRODE UND VERFAHREN ZUR HERSTELLUNG

ENZYMATIC ELECTRODE AND ITS PREPARATION METHOD

(84) Etats contractants désignés:
**AT BE CH DE FR GB IT LI LU NL SE**

(30) Priorité: **20.04.1988 FR 8805245**

(43) Date de publication de la demande:
**30.01.1991 Bulletin 1991/05**

(73) Titulaire: **Gwent Sensors Ltd**
**Pontypool, NP4 0HZ (FR)**

(72) Inventeurs:
- **EL MURR, Nabil**
  **F-56200 La Gacilly (FR)**
- **SLILAM, Mohamed**
  **F-44000 Nantes (FR)**

(74) Mandataire:
**Le Brusque, Maurice et al**
**Cabinet Harlé et Phélip**
**7, rue de Madrid**
**75008 Paris (FR)**

(56) Documents cités:

| | |
|---|---|
| **EP-A- 0 078 636** | **EP-A- 0 127 958** |
| **EP-A- 0 177 743** | **WO-A-88/07192** |
| **GB-A- 1 329 520** | **GB-A- 2 128 620** |
| **US-A- 4 224 125** | **US-A- 4 321 123** |
| **US-A- 5 272 087** | |

- **FEBS Letters, vol. 59, no. 2, November 1975, North-Holland Publishing Co., Amsterdam (NL); C. CALVOT et al., pp. 258-262#**
- **"ExocTech-Blood Glucose Test Strips", pages 1-4, 23 July 1987**
- **The Lancet 1987, 778/779**
- **Analytical Chemistry 56 (1984) 849-852**
- **Analytica Chimica Acta 182(1986) 103-112**
- **Analytica Chimica Acta 126 (1981) 23-24**
- **Phil.Trans.R.Soc.Lond.B316 (1987) 85-94**
- **Analytica Chemica Acta 228 (1990) 251-257**
- **Römpps Chemie Lexikon, 7th Edn.(1974) 2388-2389**

EP 0 409 896 B2

## Description

La présente invention a été faite au Laboratoire R.M.N. et Réactivité Chimique de l'Université de Nantes, Unité associée au Centre National de la Recherche Scientifique N. 472.

La présente invention se rapporte à une électrode enzymatique, en particulier, à une électrode à surface renouvelable par frottement ou découpe, de telle sorte que sa surface utile soit toujours identique à elle-même, quelles que soient la forme et l'application de l'électrode. L'invention s'applique également à une électrode enzymatique du type jetable. L'invention concerne également le procédé de préparation de cette électrode.

Une électrode à enzyme résulte de l'association d'un capteur électrochimique et d'une enzyme sous forme notamment immobilisée. Le fonctionnement d'une telle électrode est basé sur le principe qu'une dégradation enzymatique du substrat à doser est réalisée et que, parallèlement, l'apparition ou la disparition d'un des produits de la réaction est mesurée.

Ainsi, dans le cas de l'électrode à glucose comportant la glucose oxydase comme enzyme, la réaction enzymatique est la suivante :

$$\text{glucose} + O_2 + H_2O \xrightarrow{\text{GOD}} \text{Acide gluconique} + H_2O_2$$

Plusieurs types de détection électrochimique sont envisageables, à savoir la mesure de la disparition de $O_2$, la mesure de l'apparition des ions $H^+$ et l'oxydation anodique de $H_2O_2$ (électrode de platine) à potentiel imposé et la mesure du courant qui en résulte.

Pour immobiliser les enzymes, on connaît diverses techniques, à savoir l'inclusion, suivant laquelle l'enzyme est piégée de façon mécanique dans un polymère sous forme de gel ; la réticulation, suivant laquelle des molécules chimiques bifonctionnelles, comme le glutaraldéhyde, permettent la réticulation des molécules d'enzyme entre elles ou leur co-réticulation avec des protéines inactives, comme la gélatine et l'albumine ; le confinement, suivant lequel l'enzyme est en solution à l'intérieur d'un compartiment séparé de la solution à doser par une membrane poreuse qui ne laisse passer que les petites molécules ; et la fixation covalente qui se fait par réaction entre les groupements fonctionnels portés par une surface activée et des groupements fonctionnels de l'enzyme qui ne participent pas à l'activité catalytique de celle-ci.

Par ailleurs, la dépendance de la réaction enzymatique de la quantité d'oxygène dissous dans la solution peut être dans certains cas, en particulier pour les dosages dans les liquides biologiques, un facteur limitant de cette technique de dosage. En effet, des variations de la pression d'oxygène peuvent amener des fluctuations importantes dans les réponses de l'électrode : de même, ces réponses sont faussées lorsque la quantité d'oxygène présent devient faible. Ces inconvénients ont amené à préconiser l'utilisation de substituts à l'oxygène, notamment le ferrocène et ses dérivés, que l'on appelle médiateurs. Les réactions impliquées dans ce cas sont les suivantes dans le cas du dosage du glucose par une électrode à glucose oxydase GOD avec le ferrocène comme médiateur:

$$\text{glucose} + GOD_{[ox]} + H_2O \rightarrow \text{acide gluconique} + GOD_{[red]} \qquad [1]$$

$$GOD_{[red]} + 2[C_5H_5]_2Fe^+ \rightarrow GOD_{[ox]} + 2[C_5H_5]_2Fe + 2H^+ \text{ [2]} \qquad [2]$$

$$2[C_5H_5]_2Fe \rightleftharpoons 2[C_5H_5]_2Fe^+ + 2\,e^- \qquad [3]$$

La détection électrochimique est l'oxydation sur électrode "nue" du ferrocène en cation ferricinium (réaction 3). Le ferrocène peut alors être introduit en quantité catalytique par rapport au glucose et le courant dû à sa régénération électrochimique servira de signal de détection et de dosage du glucose.

Cette méthode a permis d'obtenir des résultats satisfaisants mais présente quelquefois l'inconvénient majeur d'introduire une substance étrangère au milieu et qui peut se révéler toxique.

Traditionnellement, dans les électrodes enzymatiques, les enzymes sont maintenues auprès de la surface de l'électrode. C'est le cas notamment de l'électrode à GOD décrite par T. IKEDA et al. dans Agric. Biol. Chem. 49(29), 541-543, 1985 et dans Agric. Biol. Chem. 50(4), 883-890, 1986, la surface de l'électrode chargée en GOD étant revêtue par un film de nitrocellulose (cf demande de brevet européen EP-A-177 743). Dans la publication "Analytical Sciences Décembre 1985, vol. 1", pages 455-457, IKEDA et al. décrivent une électrode à GOD dans laquelle la couche de GOD, appliquée sur l'électrode, est revêtue par une membrane de dialyse, une mini-grille en or et un filet de 《Nylon》.

On constate donc que, selon la technique antérieure de fixation de l'enzyme à la surface de l'électrode, on est obligé de protéger l'enzyme par un "chapeau" perméable à la solution et non à l'enzyme. On a également proposé de fixer l'enzyme sur un support chimique.

Les électrodes enzymatiques précitées permettent de faire un certain nombre de dosages spécifiques, mais elles sont souvent d'un emploi délicat, car elles demandent une préparation minutieuse avant chaque mesure et elles se saturent assez rapidement.

De plus, la surface de l'électrode peut s'abîmer facilement, et il en résulte une perte de signal et une diminution de la reproductibilité. En effet, la surface n'est pas renouvelable, sauf à effectuer à chaque fois un traitement de régénération. Par ailleurs, dans le cas de la fixation d'enzymes par réaction chimique, notamment pour la fixation de plusieurs enzymes différentes à la surface de l'électrode, il peut se produire des interférences ou des compétitions qui rendent souvent la préparation de l'électrode incontrôlable.

Selon l'invention, on propose d'emprisonner les enzymes dans une matrice conductrice. Pour les analyses, cette matrice est avantageusement contenue dans une gaine inerte, en un matériau isolant, par exemple, en verre ou en matière plastique, d'où elle émerge à une extrémité pour constituer la surface utile. Avant une mesure, il suffit, si cela s'avère nécessaire, de nettoyer sommairement l'électrode par exemple, par simple frottement sur une feuille de papier, ce qui enlève la couche superficielle usée sur quelques angströms, puis on plonge l'électrode dont la surface a ainsi été régénérée dans le milieu liquide à analyser.

L'électrode de l'invention est facile à préparer, à moindre coût, elle peut se conserver facilement, elle est toujours prête à l'emploi, elle est très maniable et fournit des résultats reproductibles. Elle peut sans inconvénient renfermer un système multienzymatique, ainsi qu'un médiateur d'électrons et/ou un coenzyme. Elle peut être aussi à usage unique et jetable.

L'électrode enzymatique selon l'invention est donc conforme à la revendication 1.

Dans FEBS Letters Vol. 59, n° 2, Novembre 1975, C. Calvot et al. décrivent la fabrication d'une membrane par mélange de lysine décarboxylase avec une solution contenant de l'albumine plasmatique et de la glutaraldhéyde, addition de particules d'oxyde de fer magnétique, coulée de la suspension sur une plaque pour obtenir une membrane d'épaisseur homogène, réticulation pendant 2 heures à la température ambiante et séparation du film formé. Ce film est fixé (par un aimant, ce qui explique la présence de particules d'oxyde de fer magnétique dans la membrane) sur une électrode à $pCO_2$ sur laquelle on peut constater la présence d'une feuille poreuse et d'une membrane perméable aux gaz. Ainsi, avec cette électrode connue, il se produit une réaction enzymatique au niveau du film, puis une diffusion du gaz carbonique, puis une détection au niveau de l'électrode. La partie enzymatique et la partie de détection sont donc indépendantes. L'idée originale qui est à la base de la présente invention est d'avoir combiné ces deux parties traditionnelles de l'électrode, ce qui permet de résoudre d'emblée l'ensemble des inconvénients présentés par ces dernières. La séparation existant dans les structures connues entre la partie enzymatique et la partie de détection pose des problèmes de perméabilité, donc de diffusion et de détection, la cinétique diffusionnelle jouant sur la reproductibilité du signal. Par ailleurs, l'utilisation d'une membrane enzymatique entraîne des difficultés liées à la réalisation de membranes à appliquer au corps de détecteur qui soient d'épaisseur constante pour assurer la diffusion correcte des produits. Enfin, il est difficile d'assurer, avec une membrane, une composition constante au cours du temps, ce qui a une influence défavorable sur le caractère répétitif des mesures.

La séparation des parties enzymatiques et de détection est également observée dans les électrodes enzymatiques décrites dans le brevet américain US-A-4 224 125 et dans la demande de brevet européen EP-A-177 743. Le document US-A-4 321 123 décrit une électrode enzymatique où l'enzyme n'est pas pris dans la masse d'électrode, mais est lié à la surface de l'électrode.

A titre d'enzyme, dans l'électrode selon l'invention, on peut utiliser n'importe quelle enzyme susceptible d'entrer en réaction avec le substrat à analyser et/ou avec toute autre substance pouvant fournir un signal électrique représentatif dudit substrat à analyser.

Les enzymes les plus courantes sont plus particulièrement les oxydoréductases qui impliquent alors l'utilisation d'un agent, dit médiateur, pour le transfert d'électrons. L'origine de l'enzyme n'est aucunement critique, car l'électrode de l'invention ne fait appel qu'à sa propriété de réaction directement ou indirectement détectable par voie électrochimique.

Il est également possible d'utiliser, conjointement à une enzyme donnée, son co-enzyme, lequel est lui-même détectable par voie électrochimique. C'est le cas par exemple des différentes déshydrogénases.

La quantité d'enzyme présente dans la matrice conductrice peut varier dans de larges limites, en fonction de l'activité propre de chaque enzyme. Plus l'activité de l'enzyme est élevée, moins la quantité en poids de l'enzyme est grande dans la matrice, étant observé que des proportions excessives d'enzyme ne nuisent pas à la mesure proprement dite mais sont inutilement coûteuses. Mais il va sans dire que la quantité d'enzyme minimale doit être telle qu'elle fournisse un signal de mesure pratiquement exploitable. Par exemple, pour l'électrode à glucose oxydase (GOD), les proportions peuvent varier de 0,4 à 15% en poids avec une enzyme à UI = 200, et plus particulièrement de 1 à 5% en poids.

La granulométrie de la poudrer conductrice n'est pas critique ; on a obtenu de bons résultats avec une poudre de granulométrie comprise entre 1 um et 25 um, par exemple de 5 um en moyenne.

La poudre conductrice est choisie notamment parmi les poudres de carbone ou les poudres de graphite.

De préférence, la matrice est formée par le mélange de la poudre avec un liant inerte, c'est-a-dire ne réagissant pas dans le domaine de potentiel considéré, et utilisé notamment à raison de 10 à 40 % en poids par rapport à la matrice conductrice pâteuse ainsi obtenue. Le liant est avantageusement un liant hydrophobe ; l'huile de paraffine, l'alphabromonaphtalène et l'huile de silicone, ont donné de bons résultats. Pour le choix du liant, on pourra se reporter à R.N. ADAMS, dans "Electrochemistry at solid electrodes", Ed. Marcel DEKKER, New York, 1969. Dans certains cas, le liant n'est pas indispensable, par exemple si la poudre de carbone est à l'état matriçable.

La quantité de liant est choisie de telle sorte que la matrice ne soit pas trop fluide par excès de liant, et, qu'à l'inverse, elle ne s'effrite pas par défaut de liant.

Par ailleurs, l'enzyme est immobilisée au sein de la matrice de matière conductrice par l'intermédiaire d'au moins un agent réticulant ou bien par emprisonnement physique dans la matrice constitutive de l'électrode par exemple du carbone. Comme agent réticulant, on peut mentionner le glutaraldéhyde.

Selon l'invention, on peut également prévoir que la matrice de matière conductrice renferme en outre au moins un agent médiateur en une quantité de 0,01 à 3 % en poids, en particulier de 0,05 à 1% en poids, par rapport à ladite matrice, ce médiateur étant choisi notamment parmi le ferrocène, le nickelocène et leurs dérivés, ainsi que la benzoquinone et autres agents de transfert d'électrons.

A titre illustratif, les dérivés du ferrocène, décrits à titre de médiateurs en solution par A.E.G. GASS et al, dans "Anal. Chem. 1984, 56, 667-671, peuvent être incorporés dans la matrice de l'invention.

La matrice de l'électrode selon l'invention peut également renfermer au moins un co-enzyme.

Pour préparer l'électrode enzymatique selon l'invention, dans un premier mode de réalisation, on mélange intimement :

- une pâte ou matrice homogène résultant du mélange intime d'une poudre conductrice avec un liant ;
- au moins une enzyme ;
- une solution de réticulation de l'enzyme ; et dans un second mode de réalisation, on mélange intimement une poudre conductrice matriçable et au moins une enzyme et on soumet ce mélange à des efforts mécaniques, en particulier de compression, pour obtenir une matrice solide emprisonnant physiquement l'enzyme.

Dans les deux cas, on peut incorporer à la pâte ou matrice ou au mélange un médiateur et/ou un co-enzyme.

Dans le cas où la pâte ou matrice ou le mélange renferment un médiateur, on peut préparer une fraction de poudre conductrice renfermant de façon homogène le médiateur, en mélangeant ce dernier en solution organique avec la poudre conductrice, puis en faisant évaporer le solvant, et on mélange cette fraction à la matrice conductrice renfermant l'enzyme.

Pour les dosages, le montage utilisant l'électrode de l'invention est un montage classique, l'une des extrémités de l'électrode étant mise en contact avec la solution dans la cellule de mesure, et l'autre, avec le circuit électrique du potentiostat.

L'électrode enzymatique selon l'invention peut revêtir différentes formes, entre autres une forme portable à usage renouvelé ou à usage unique.

L'invention sera illustrée sans être aucunement limitée par la description qui suit, faite en référence aux dessins annexés et aux exemples suivants, dans lesquels les pourcentages sont donnés en poids, sauf indication contraire :

- Les figures 1 à 19 sont des diagrammes montrant les résultats des dosages et mesures réalisés avec des électrodes selon l'invention : et
- La figure 20 illustre schématiquement une électrode conforme à un mode de réalisation de l'invention, cette électrode ayant une forme générale tubulaire.

L'électrode 1 est représentée de façon schématique sur la figure 20. Elle se compose d'un porte-électrode 2 à une extrémité duquel est disposée une fenêtre de lecture 3 et se terminant à l'autre extrémité par un contact 4. Au voisinage de ce contact, le porte-électrode présente un plus petit diamètre, cette partie 5 de plus petit diamètre portant un filetage 6. La matrice 7 selon l'invention renfermant l'enzyme est logée dans une gaine cylindrique 8, de façon à émerger à l'une des extrémités de ladite gaine 8 pour constituer la surface utile 9 de l'électrode. La gaine 8 se prolonge, à l'extrémité opposée, au-delà de la matrice 7 pour constituer une partie 10 portant un filetage 11, destiné à coopérer avec le filetage 6 du porte-électrode 2, le contact 4 étant constamment en appui sur la face 9a de la matrice 7 opposée à la surface 9.

Par ailleurs, au voisinage de sa bordure associée à cette surface 9, la gaine 8 comporte un filetage externe

12 destiné à coopérer avec le taraudage 13 de la jupe périphérique 14 d'un capuchon 15 sur le fond 16 duquel est appliquée une pastille 17 de papier filtre ou feutre ou matière analogue. Une électrode auxiliaire et/ou de référence 18 est associée à l'électrode enzymatique 7.

Pour l'utilisation, le capuchon 15 est dévissé. Après chaque utilisation, on replace le capuchon 15 et pour régénérer la surface utile 9, on peut faire tourner légèrement le porte-électrode 2, ce qui assure le frottement de cette surface 9 contre la pastille 17.

Exemple 1: Dosage du glucose à l'aide d'une électrode à pâte de carbone contenant de la glucose oxydase, en présence de l'acide monocarboxylique du ferrocène comme médiateur, dans le milieu d'analyse.

(a) Préparation de l'électrode

On mélange 1 g d'une poudre de carbone de granulométrie de valeur moyenne de 5 μm (Carbone Lorraine) et 0,36 ml d'huile de paraffine jusqu'à obtention d'une pâte homogène.

On mélange intimement 300 mg de la pâte de carbone ainsi obtenue avec 0,3 ml d'une solution de réticulation ayant la formulation suivante :

| | |
|---|---|
| Glutaraldéhyde à 2,5 % | 25% |
| Albumine bovine à 15% | 29% |
| Solution tampon phosphate pH 6,8 | 56% |

ainsi qu'avec la quantité choisie de glucose oxydase. On laisse reposer le mélange ainsi obtenu dans le mortier, à 4°C, pendant 10 à 30 minutes. On gratte pour obtenir une sorte de poudre que l'on reprend avec 0,06 ml d'huile de paraffine.

(b) Dosage du glucose

Ce dosage est conduit en présence de l'acide monocarboxylique du ferrocène comme médiateur, les réactions mises en jeu étant analogues à celles indiquées dans le préambule de la présente description pour le ferrocène.

Le milieu d'analyse est un milieu tampon phosphate à pH 8-9.

La Figure 1(a) montre la forme du signal obtenu en voltamétrie cyclique avec cette électrode lorsque l'acide monocarboxylique du ferrocène est seul en solution. La courbe $i = f(E)$ ainsi obtenue correspond à l'oxydation dudit acide en le cation ferrocénique correspondant.

La Figure 1(b) est obtenue lorsque du glucose est ajouté à la solution précédente. On note une augmentation du courant d'oxydation en présence du glucose.

Il faut signaler qu'aucun courant n'est détecté à ce potentiel avec l'électrode lorsque le médiateur est absent de la solution, même si la solution contient du glucose.

Sur la Figure 2, on a tracé les courbes de réponse $i = f(C)$ de cette électrode en fonction de la concentration en glucose, pour différentes concentrations en GOD dans la pâte de carbone de l'électrode, dans les conditions suivantes :

. Concentration en médiateur dans le milieu d'analyse: 0,5 x $10^{-3}$ M
. pH : 8,9.
. vitesse de balayage : 2 mV/s

Légende de la Figure 2

Abscisses : concentration du glucose en mM
Ordonnées : intensité du courant en μA

| Courbe | Nombre d'unités de GOD x $10^{-3}$ par gramme de pâte de carbone |
|---|---|
| (a) | 1,4 |
| (b) | 2,8 |
| (c) | 4,3 |
| (d) | 5,8 |

La Figure 2 montre la linéarité de la réponse de l'électrode en fonction de la concentration de glucose. Les quatre droites de cette figure montrent que, plus la concentration en GOD dans la pâte de l'électrode est grande, plus le signal est grand, c'est-à-dire plus la détection est sensible.

Sur la Figure 3, on a tracé les courbes de réponse i = f(C) de cette électrode en fonction de la concentration en médiateur, pour différentes concentrations en glucose, dans les conditions suivantes :

. Concentration en GOD dans la pâte : 1,5 % en poids ($10^3$ U par gramme de pâte)
. pH : 8,8
. vitesse de balayage : 2 mV/s

<u>Légende de la Figure 3</u>

Abscisses : concentration en médiateur dans le milieu d'analyse en mM
Ordonnées : intensité du courant en µA

| Courbe | Concentration en glucose en $10^{-3}$ M |
|---|---|
| (a) | 0 |
| (b) | 6 |
| (c) | 12 |
| (d) | 18 |
| (e) | 21 |
| (f) | 30 |

La Figure 3 montre que la sensibilité du signal est d'autant plus grande que la concentration de la solution en médiateur est grande.

<u>Exemple 2</u>: Dosage du glucose à l'aide d'une électrode à pâte de carbone contenant de la glucose oxydase et de la p-ferrocenyl aniline comme médiateur.

(a) <u>Préparation de l'électrode</u>

On dissout une quantité choisie de p-ferrocenyl aniline $(C_5H_5)Fe(C_5H_4-C_6H_4-NH_2)$ dans du chlorure de méthylène, et à la solution obtenue, on ajoute une quantité déterminée de poudre de carbone, analogue à celle de l'Exemple 1. On fait évaporer le chlorure de méthylène tout en agitant la mélange. Après évaporation totale du solvant, on obtient une poudre de carbone contenant d'une façon homogène le médiateur.

Deux méthodes peuvent être utilisées pour préparer la pâte servant d'électrode :

(a) On mélange 1 g de cette poudre avec 0,36 ml d'huile de paraffine jusqu'à obtention d'une pâte homogène, puis on mélange intimement 300 mg de la pâte de carbone ainsi obtenue avec 300 mg de la pâte contenant GOD préparée selon l'Exemple 1.
(b) On prépare une poudre contenant l'enzyme en mélangeant par exemple 1 g de poudre de carbone avec 1 ml de la solution de réticulation contenant l'enzyme selon l'Exemple 1 et on laisse à 4°C pour 10 à 30 minutes, puis on gratte jusqu'à l'obtention de poudre fine. La pâte pour l'électrode est alors préparée en mélangeant par exemple 1 g de cette poudre avec 1 g de la poudre contenant le médiateur et 0,72 ml d'huile de paraffine.

(b) <u>Dosage du glucose</u>

Les réactions mises en jeu sont analogues à celles indiquées à l'Exemple 1. Le milieu d'analyse est un milieu tampon phosphate à pH ~ 8,8.
La Figure 4(a) montre la forme du signal obtenu $i = f(E)$ , en voltamétrie cyclique, en milieu tampon précité et en l'absence de glucose, avec l'électrode contenant GOD et le médiateur. La Figure 4(b) montre ce même signal après addition de glucose.
Sur la Figure 5, on a tracé la courbe $1/R = f(1/C)$ , R étant étant le rapport du signal obtenu en présence de glucose à celui obtenu en l'absence de glucose, et C, la concentration en glucose exprimée en mM. Les conditions étaient les suivantes :

. quantité de médiateur dans la pâte : 0,4 % ;
. quantité de GOD dans la pâte : $7.10^3$ U/g ;
. vitesse de balayage : 2 mV/s

Une corrélation linéaire est obtenue entre 1/R et 1/C.
On a également obtenu des relations linéaires entre 1/R et 1/C en faisant varier la concentration du médiateur et celle de l'enzyme dans l'électrode.
Sur la Figure 6, on a tracé la courbe qui relie l'amplitude du signal i en µA à la concentration, en mMole, de la solution en glucose. Sur la Figure 7, on a tracé la partie linéaire de cette courbe, c'est-à-dire, pour des concentrations en glucose égales ou inférieures à $10^{-2}$ mole.$1^{-1}$.
Les conditions dans ces deux cas étaient les suivantes :

. quantité de médiateur dans la pâte : 0,46 % ;
. quantité de GOD dans la pâte : $5,15.10^3$ U/g ;
. vitesse de balayage : 2 mV/s
. solution tampon phosphate 0,1 M ; pH = 7

Sur la Figure 8, on a tracé la courbe représentant la variation de R en fonction de la concentration en médiateur (exprimée en % par rapport à la totalité de la pâte). Les conditions étaient les suivantes :

. concentration en glucose dans le milieu d'analyse : $20 \times 10^{-3}$ M
. concentration en GOD dans l'électrode : 2,9 % (soit $6,25 \times 10^3$ U/g)
. pH = 8,9
. vitesse de balayage : 2 mV/s

Cette courbe montre que, bien qu'un important signal soit détecté pour toutes les concentrations en médiateur (entre 0 et 0,6%), celui-ci passe par un maximum pour des concentrations se situant aux alentours de 0,15%.
Sur la Figure 9, on a tracé la courbe représentant la variation de R en fonction de la concentration en GOD dans l'électrode (exprimée en U GOD/g de pâte), pour des concentrations en médiateur de 0,15 et 0,2% (courbes respectivement a et b) par rapport à la totalité de la pâte, dans les conditions suivantes :

. concentration en glucose fixée à $20 \times 10^{-3}$ M ;
. pH = 8,9 ;
. vitesse de balayage : 2 mV/s.

On note également ici qu'un signal optimum est obtenu lorsque l'électrode contient environ $5 \times 10^3$ Unités de GOD/g. Le signal reste cependant toujours important et exploitable pour des concentrations en-deçà et au-delà de

cette valeur.

Exemple 3: Dosage du galactose à l'aide d'une électrode à pâte de carbone contenant de la galactose oxydase et de la p-ferrocenyl aniline.

(a) Préparation de l'électrode

On procède comme à l'exemple 2(a), en remplaçant la GOD par la galactose oxydase.

(b) Dosage du galactose

La Figure 10(a) montre la forme du signal obtenu en voltamétrie cyclique i = f(E) avec cette électrode dans un milieu tampon phosphate à pH ~ 8,9, en l'absence de galactose, et la Figure 10(b) montre ce même signal après l'addition de galactose.

Exemple 4: Dosage de la L-leucine à l'aide d'une électrode à pâte de carbone contenant de la L-acide amino oxydase et de la p-ferrocenyl aniline.

(a) Préparation de l'électrode

On procède comme à l'Exemple 2(a), en remplaçant la GOD par de la L acide amino oxydase.

(b) Dosage de la L-leucine. La Figure 11(a) montre la forme du signal obtenu avec cette électrode dans un milieu tampon phosphate à pH ~ 8,9, en l'absence de L-leucine, et la Figure 11(b) montre l'effet de l'addition de L-leucine.

Exemple 5: Dosage du glucose à l'aide d'une électrode à pâte de carbone contenant de la glucose oxydase et du nickelocène.

(a) Préparation de l'électrode

On procède comme à l'Exemple 2(a), en remplaçant la p-ferrocenyl aniline par le nickelocène: $(C_5H_5)_2Ni$.

(b) Dosage du glucose

La Figure 12 montre le signal obtenu avec cette électrode dans un milieu tampon phosphate à pH ~ 8,9, en l'absence de glucose (courbe a) et après addition de glucose (courbe b).

Exemple 6: Dosage du glucose à l'aide d'une électrode à pâte de carbone contenant de la glucose oxydase et de la benzoquinone.

(a) Préparation de l'électrode

On procède comme à l'Exemple 2 (a), en remplaçant le p-ferrocenyl aniline par la benzoquinone.

(b) Dosage du glucose.

La Figure 13 montre la forme du signal obtenu avec cette électrode dans un milieu tampon phosphate à pH ~ 8,9, en l'absence de glucose (courbe a) et après addition de glucose (courbe b).

Exemple 7: Dosage du saccharose à l'aide d'une électrode à pâte de carbone contenant de l'invertase et de la glucose oxydase, et de l'acide monocarboxylique du ferrocène comme médiateur en solution.

(a) Préparation de l'électrode

On procède comme à l'Exemple 1, avec utilisation de 20 mg de GOD/(6300 U/g de pâte) et 20 mg d'invertase (24000 U/g de pâte), la quantité totale de poudre de carbone étant de 300 mg.

(b) Dosage du saccharose

La Figure 14 montre la forme du signal obtenu avec cette électrode dans un milieu tampon phosphate à pH ~ 8,9, en l'absence de saccharose (courbe a) et après addition de saccharose (courbe b).
Sur la Figure 15, on a tracé les variations de $I - I_0$ en fonction de la concentration en saccharose (exprimée en mM), dans les conditions suivantes :

. milieu tampon phosphate pH ~ 8,9 ;
. vitesse de balayage : 2 mV/s

La relation entre le signal et la concentration en saccharose est une relation linéaire.

Exemple 8: Dosage du saccharose à l'aide d'une électrode à pâte de carbone contenant de l'invertase, de la glucose oxydase et de la p-ferrocenyl aniline.

(a) Préparation de l'électrode

On procède comme à l'Exemple 2, avec les concentrations suivantes :

. GOD : 300 U/g de pâte
. Invertase : 28 000 U/g de pâte
. p-Ferrocenyl aniline : 0,1% en poids de pâte.

(b) Dosage du saccharose

La Figure 16 montre la forme du signal obtenu avec cette électrode dans un milieu tampon phosphate à pH ~ 8,9, en l'absence de saccharose (courbe a) et après addition de saccharose (courbe b).
Sur la Figure 17, on a tracé les variations de I en fonction de la concentration en saccharose (exprimée en mM), dans les conditions suivantes :

. milieu tampon phosphate pH ~ 8,9 ;
. vitesse de balayage : 2 mV/s

La relation entre le signal et la concentration en saccharose est une relation linéaire.
Le dosage du saccharose peut être avantageusement réalisé à l'aide d'une électrode à trois enzymes en incorporant à la pâte décrite en (a) la mutarotase qui aura pour rôle de déplacer l'équilibre existant entre les anomères $\alpha$ et $\beta$ du glucose (formé grâce a l'invertase) au profit de ce dernier qui sera alors directement détecté par l'électrode. Une telle électrode à trois enzymes présente alors un signal plus important que celui donné par l'électrode a deux enzymes et permet donc de détecter des concentrations très faibles en saccharose.

Exemple 9: Dosage de l'éthanol à l'aide d'une électrode à pâte de carbone contenant de l'alcool déshydrogénase et $NAD^+$.

(a) Préparation de l'électrode

L'électrode est préparée comme à l'Exemple 2(a), sauf que $NAD^+$ remplace le médiateur, la quantité choisie de $NAD^+$ étant dissoute dans l'eau puis ajoutée à de la poudre de carbone.

(b) Dosage de l'éthanol

La Figure 18 montre le signal obtenu avec cette électrode dans un milieu tampon phosphate à pH ~ 8,9, en l'absence d'éthanol (courbe a) et après addition d'éthanol (courbe b). Sur la Figure 19, on a tracé la courbe représentant la variation de I en fonction de la concentration (v/v) en éthanol/eau. Les conditions sont les suivantes :

. milieu tampon phosphate pH ~ 8,9
. vitesse de balayage 50 mV/s

La relation est une relation linéaire.

L'électrode enzymatique selon l'invention peut être utilisée dans tous les domaines mettant en jeu des réactions enzymatiques, en tirant profit de leur sélectivité, par exemple, pour les dosages dans l'industrie chimique, dans les domaines médicaux et biologiques, ainsi que dans les industries agro-alimentaires.

## Revendications

1. Electrode enzymatique présentant une surface renouvelable par frottement ou découpe, et étant directement en contact avec le substrat à doser sans membrane intermédiaire perméable ou semi-perméable, comprenant une partie enzymatique et une partie de détection combinées et reliées à un circuit électrique, ladite électrode consistant en une matrice réalisée à partir d'une poudre conductrice assurant la détection et renfermant, en mélange intime avec ladite poudre, au moins une enzyme immobilisée, ou une enzyme immobilisée et un agent médiateur ou un coenzyme, incorporée de façon homogène dans la matrice, le médiateur et/ou le coenzyme n'étant pas réticulés à la matrice, ladite matrice étant contenue dans une gaine inerte d'où elle émerge à une extrémité pour constituer la surface utile de l'électrode, un contact du circuit électrique de l'électrode étant constamment en appui sur l'autre extrémité de la matrice.

2. Electrode selon la revendication 1, caractérisée par le fait que la poudre conductrice est de la poudre de carbone ou de la poudre de graphite.

3. Electrode selon l'une des revendications 1 et 2, caractérise par le fait que la matrice est formée par le mélange intime de la poudre avec un liant inerte utilisé notamment à raison de 10 à 40% en poids par rapport à la pâte conductrice.

4. Electrode selon la revendication 3, caractérisée par le fait que le liant est un liant hydrophobe.

5. Electrode selon la revendication 4, caractérisée par le fait que le liant est constitué par de l'huile de paraffine, de l'alphabromonaphtalène ou une huile de silicone.

6. Electrode selon l'une des revendications 1 à 5, caractérisée par le fait que l'enzyme est fixée au sein de la matrice conductrice par l'intermédiaire d'au moins un agent réticulant.

7. Electrode selon la revendication 6, caractérisée par le fait que l'agent réticulant est le glutaraldéhyde.

8. Electrode selon l'une des revendications 1 à 7 , caractérisée par le fait qu'elle comprend une poudre conductrice matriçable emprisonnant physiquement l'enzyme.

9. Electrode selon l'une des revendications 1 à 8, caractérisée par le fait qu'elle contient plusieurs enzymes immobilisées dans la matrice.

10. Electrode selon l'une des revendications 1 à 9, caractérisée par le fait que le médiateur est choisi parmi le ferrocène, le nickelocène et leurs dérivés, ainsi que la benzoquinone, et autres agents de transfert d'électrons.

11. Electrode enzymatique selon l'une des revendications 1 à 10 pour le dosage du glucose, caractérisée par le fait que l'enzyme est la glucose oxydase et que la matrice conductrice renferme en outre un médiateur choisi parmi la p-ferrocenyl aniline, le nickelocène et la benzoquinone.

12. Electrode selon l'une des revendications 1 à 10, pour le dosage du saccharose, caractérisée par le fait que les enzymes immobilisées sont l'invertase et la glucose oxydase, ou bien l'invertase, la glucose oxydase et la mutarotase.

13. Electrode selon l'une des revendications 1 à 10, pour le dosage de l'éthanol, caractérisée par le fait que l'enzyme est l'alcool déshydrogénase qui est associée à $NAD^+$.

14. Procédé de préparation de l'électrode enzymatique telle que définie à l'une des revendications 1 à 13 caractérisée par le fait qu'on mélange intimement:

    - une pâte ou matrice homogène résultant du mélange intime d'une poudre conductrice avec un liant;
    - au moins une enzyme;

- une solution de réticulation de l'enzyme,
- puis on incorpore un agent médiateur et/ou un coenzyme.

**15.** Procédé selon la revendication 14, caractérisé en ce qu'on mélange intimement une poudre conductrice matriçable et au moins une enzyme avec un agent médiateur et/ou un coenzyme et qu'on soumet ce mélange à des efforts mécaniques, en particulier de compression, pour obtenir une matrice solide emprisonnant physiquement l'enzyme.

**Claims**

**1.** Enzymatic electrode having a surface which is renewable by friction or cutting, and being directly in contact with the substrate to be determined without intermediate permeable or semi-permeable membrane, comprising an enzymatic part and a detection part combined and connected to an electric circuit, said electrode consisting of a matrix made from a conducting powder providing for the detection and containing, intimately mixed with said powder, at least one immobilized enzyme, or one immobilized enzyme and a mediator agent or a coenzyme, homogeneously incorporated in the matrix, the mediator and/or the coenzyme not being cross-linked with the matrix, said matrix being embodied in an inert sheath from which it protrudes at one end to form the useful surface of the electrode, a contact of the electric circuit continuously resting on the other end of the matrix.

**2.** Electrode according to claim 1, characterized in that the conducting powder is carbon powder or graphite powder.

**3.** Electrode according to any one of claims 1 and 2, characterized in that the matrix is formed by the intimate mixture of the powder with an inert binder used, in particular, in a proportion of 10 to 40 % by weight relative to the conducting paste.

**4.** Electrode according to claim 3, characterized in that the binder is a hydrophobic binder.

**5.** Electrode according to claim 4, characterized in that the binder consists of paraffin oil, alphabromonaphthalene or a silicon oil.

**6.** Electrode according to any one of claims 1 to 5, characterized in that the enzyme is bound inside the conducting matrix by means of at least one cross-linking agent.

**7.** Electrode according to claim 6, characterized in that the cross-linking agent is glutaraldehyde.

**8.** Electrode according to any one of claims 1 to 7, characterized in that it comprises a conducting powder capable of forming a matrix physically imprisoning the enzyme.

**9.** Electrode according to any one of claims 1 to 8, characterized in that it contains several enzymes immobilized in the matrix.

**10.** Electrode according to any one of claims 1 to 9, characterized in that the mediator is selected from ferrocene, nickelocene and derivatives thereof, as well as from benzoquinone and other electron transfer agents.

**11.** Electrode according to any one of claims 1 to 10, for the determination of glucose, characterized in that the enzyme is glucose oxidase and in that the conducting matrix further embodies a mediator selected from p-ferrocenylaniline, nickelocene and benzoquinone.

**12.** Electrode according to any one of claims 1 to 10, for the determination of sucrose, characterized in that the immobilized enzymes are invertase and glucose oxidase or invertase, glucose oxidase and mutarotase.

**13.** Electrode according to any one of claims 1 to 10, for the determination of ethanol, characterized in that the enzyme is alcohol deshydrogenase which is combined with $NAD^+$.

**14.** Process for the preparation of the enzymatic electrode as defined in any one of claims 1 to 13, characterized in that the following are intimately mixed:

- a homogenous paste or matrix resulting from the intimate mixture of a conducting powder with a binder ;
- at least one enzyme ;

- an enzyme cross-linking solution ;
- then a mediator agent and/or a coenzyme is incorporated.

**15.** Process according to claim 14, characterized in that a conducting paste capable of forming a matrix and at least one enzyme are intimately mixed with a mediator agent and/or a coenzyme and in that this mixture is subjected to mechanical forces, in particular, compression, in order to provide a solid matrix physically imprisoning the enzyme.

**Patentansprüche**

**1.** Enzymatische Elektrode mit einer durch Reibung oder Abschneiden erneuerbaren Oberflache, wobei die Elektrode direkt mit einem zu bestimmenden Substrat in Kontakt steht, ohne dazwischenliegende permeable oder semipermeable Membran, und mit einem enzymatischen Teil und einem analytischen Teil, die kombiniert sind und mit einem Stromkreis verbunden sind, wobei die Elektrode aus einer Matrix besteht, die aus einem leitenden Pulver hergestellt ist, das den Nachweis sicherstellt und innig mit dem Pulver vermischt wenigstens ein bewegungsunfähiges Enzym, oder ein bewegungsunfähiges Enzym und einen Überträger oder ein Coenzym, in homogener Form in die Matrix eingebracht einschliesst, wobei der Überträger und/oder das Coenzym nicht kovalent an die Matrix gebunden sind, wobei die Matrix in einer inerten Hülle enthalten ist, aus der sie mit einem Ende herausragt, um die brauchbare Oberfläche der Elektrode zu bilden und wobei ein Kontakt des Stromkreises ständig am anderen Ende anliegt.

**2.** Elektrode nach Anspruch 1, dadurch gekennzeichnet, daß das leitende Pulver Kohlenstoffpulver oder Graphitpulver ist.

**3.** Elektrode nach Anspruch 1 order 2, dadurch gekennzeichnet, daß die Matrix gebildet wird durch inniges Vermischen des Pulvers mit einem inerten Bindemittel, das besonders im Verhältnis von 10 bis 40 Gew.-% bezogen auf die leitende Paste eingesetzt wird.

**4.** Elektrode nach Anspruch 3, dadurch gekennzeichnet, daß das Bindemittel ein hydrophobes Bindemittel ist.

**5.** Elektrode nach Anspruch 4, dadurch gekennzeichnet, daß das Bindemittel aus einem Paraffinöl, aus $\alpha$-Bromnaphthalin oder aus einem Siliconöl besteht.

**6.** Elektrode nach einem der Ansprüche 1 bis 5, dadurch gekennzeichnet, daß das Enzym mit Hilfe wenigstens eines Vernetzungsmittels innerhalb der Matrix aus leitendem Material fixiert ist.

**7.** Elektrode nach Anspruch 6, dadurch gekennzeichnet, daß das Vernetzungsmittel Glutaraldehyd ist.

**8.** Elektrode nach einem der Ansprüche 1 bis 7, dadurch gekennzeichnet, daß sie ein leitendes, zu einer Matrix formbares Pulver umfaßt, welches das Enzym physikalisch einschließt.

**9.** Elektrode nach einem der Ansprüche 1 bis 8, dadurch gekennzeichnet, daß sie in der Matrix mehrere bewegungsunfähige Enzyme enthält.

**10.** Elektrode nach einem der Ansprüche 1 bis 9, dadurch gekennzeichnet, daß der Überträger augsgewählt ist aus Ferrocen, Nicleocen und deren Derivaten, sowie aus Benzochinon und anderen Elektronon-Übertragungsreagenzien.

**11.** Elektrode nach einem der Ansprüche 1 bis 10, zur Bestimmung von Glucose, dadurch gekennzeichnet, daß das Enzym Glucoseoxidase ist, und daß die leitende Matrix außerdem einen Überträger einschließt, ansgewählt aus p-Ferrocenylanilin, Nickelocen und Benzochinon.

**12.** Elektrode nach einem der Ansprüche 1 bis 10 zur Bestimmung vos Saccharose, dadurch gekennzeichnet, daß die bewegungsunfähigen Enzyme Invertase und Glucoseoxidase oder aber Invertase, Glucoseoxidase und Mutarotase sind.

**13.** Elektrode nach einem der Ansprüche 1 bis 10 zur Bestimmung von Ethanol, dadurch gekennzeichnet, daß das Enzym an $NAD^+$ assoziierte Alkoholdehydrogenase ist.

**14.** Verfahren zur Herstellung der enzymatischen Elektrode nach einem der Ansprüche 1 bis 13, dadurch gekennzeichnet, daß:

- eine homogene Paste oder Matrix, entstanden aus inniger Vermischung eines leitenden Pulvers mit einem Bindemittel,
- wenigstens ein Enzym und
- eine Lösung zur Vernetzung des Enzyms
- innig vermischt werden
- Übertragungsreagens und/oder ein Coenzym zugegeben wird.

**15.** Verfahren nach Anspruch 14, dadurch gekennzeichnet, daß ein leitendes, zu einer Matrix formbares Pulver und wenigstens ein Enzym mit einem Übertragungsreagens und/oder einem Coenzym innig vermischt werden, und daß diese Mischung mechanischer Einwirkung, insbesondere Druck ausgesetzt wird, um eine feste Matrix zu erhalten, die das Enzym physikalisch einschließt.

FIG.1

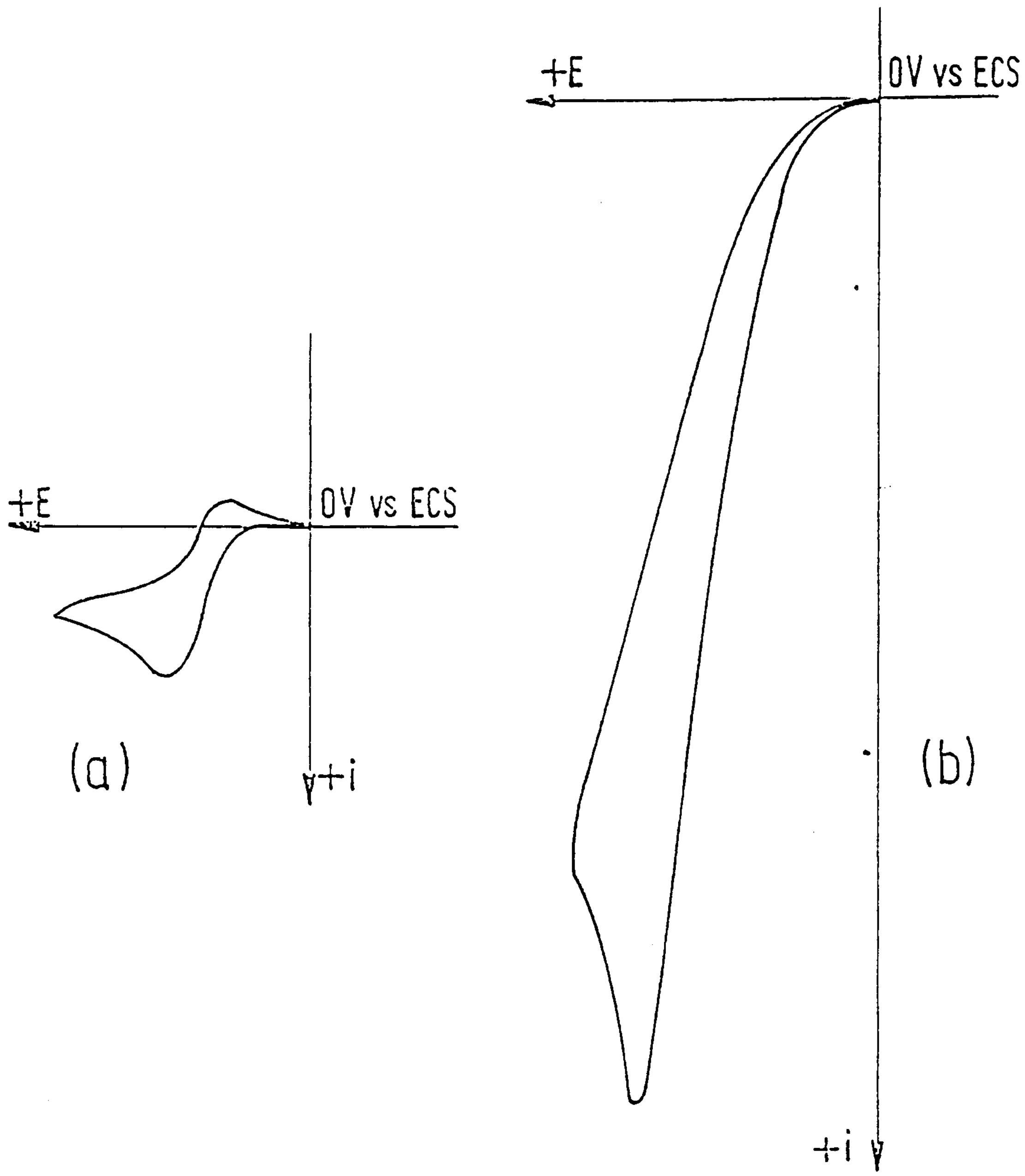

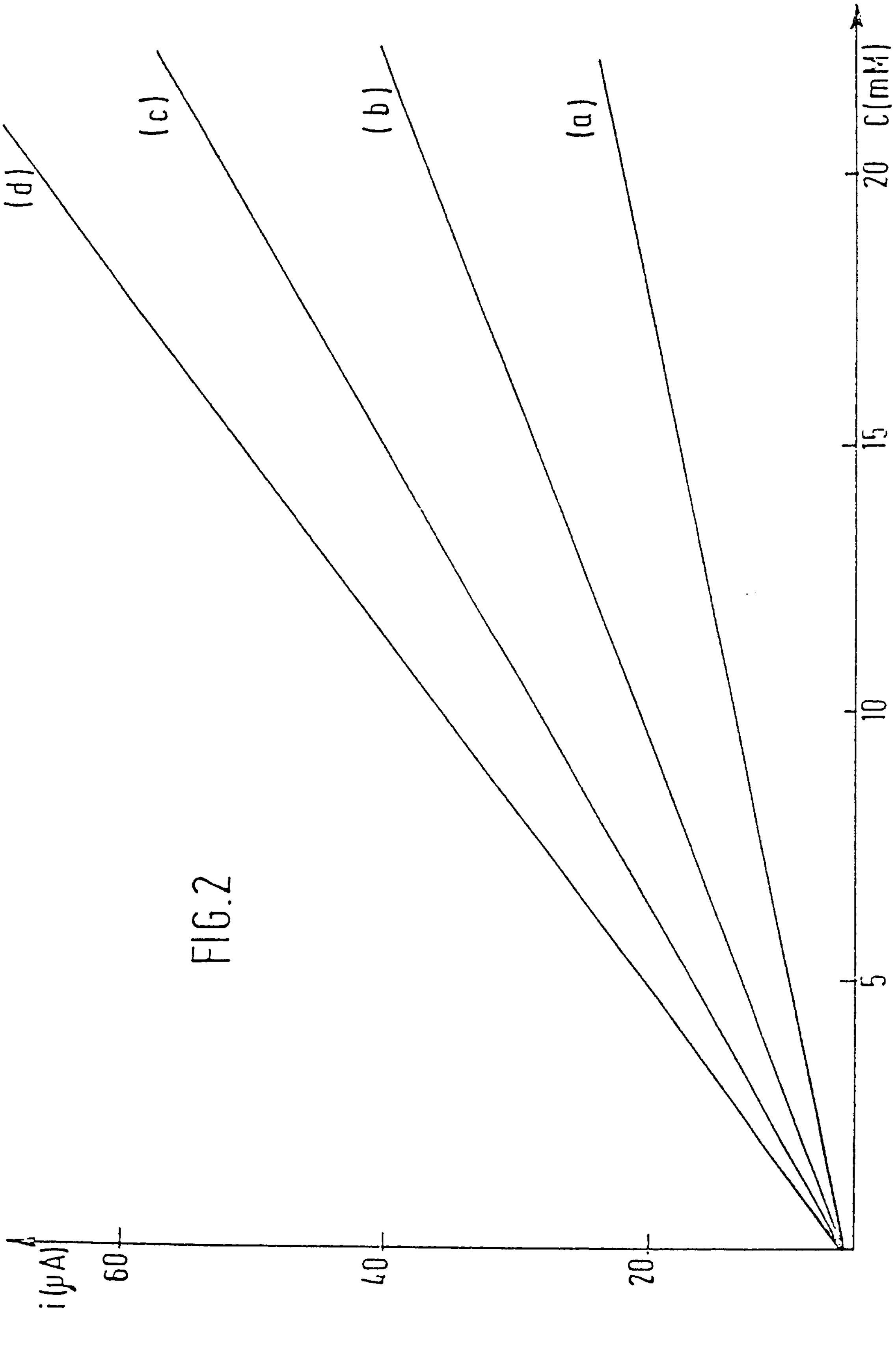


FIG.2

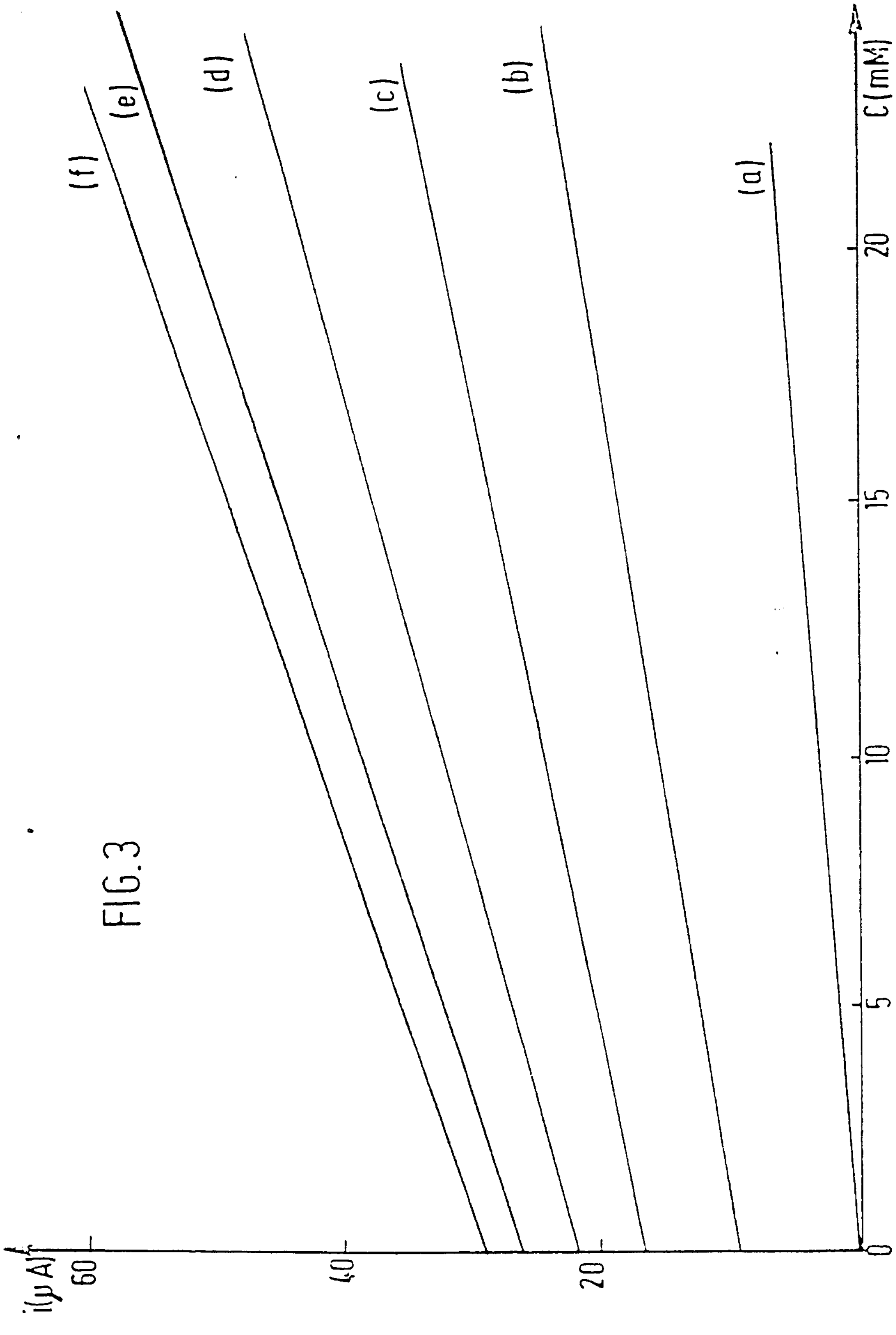
FIG.3
i(μA)
60
40
20
0
5
10
15
20
C(mM)
(a)
(b)
(c)
(d)
(e)
(f)

FIG.4

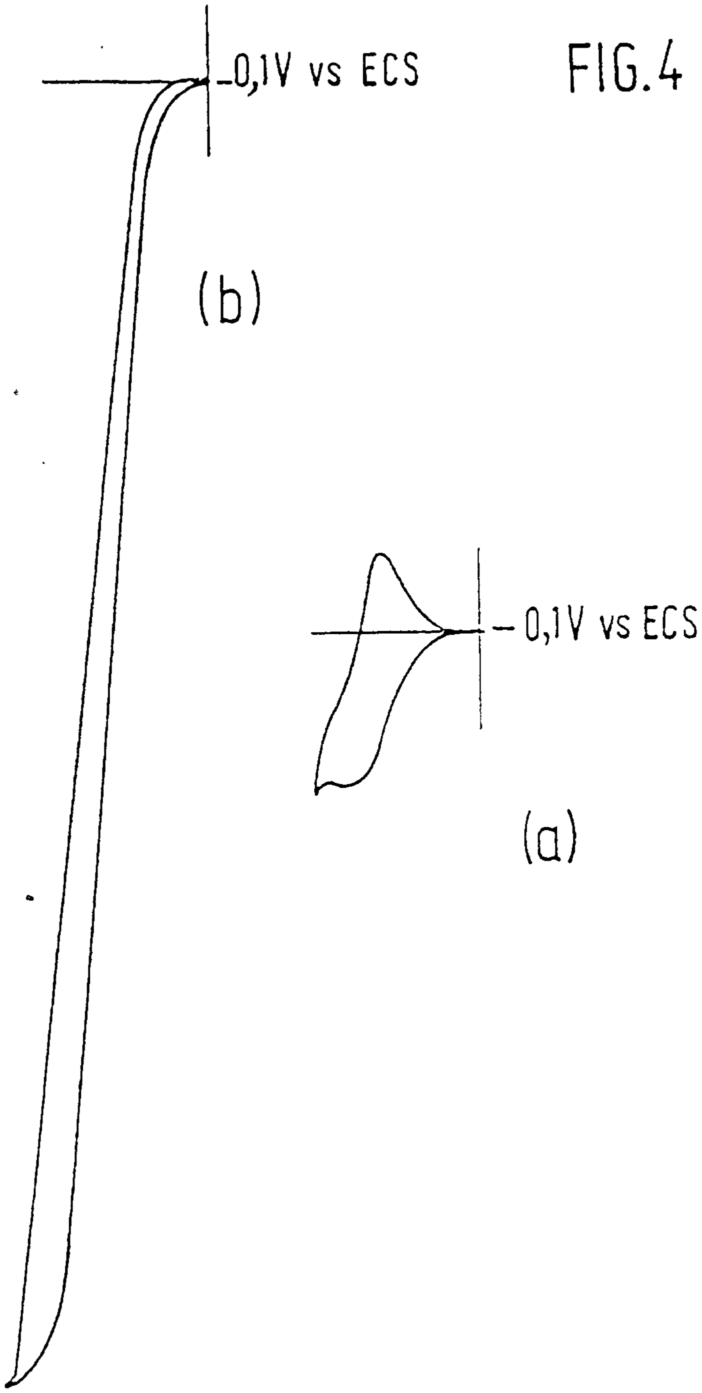

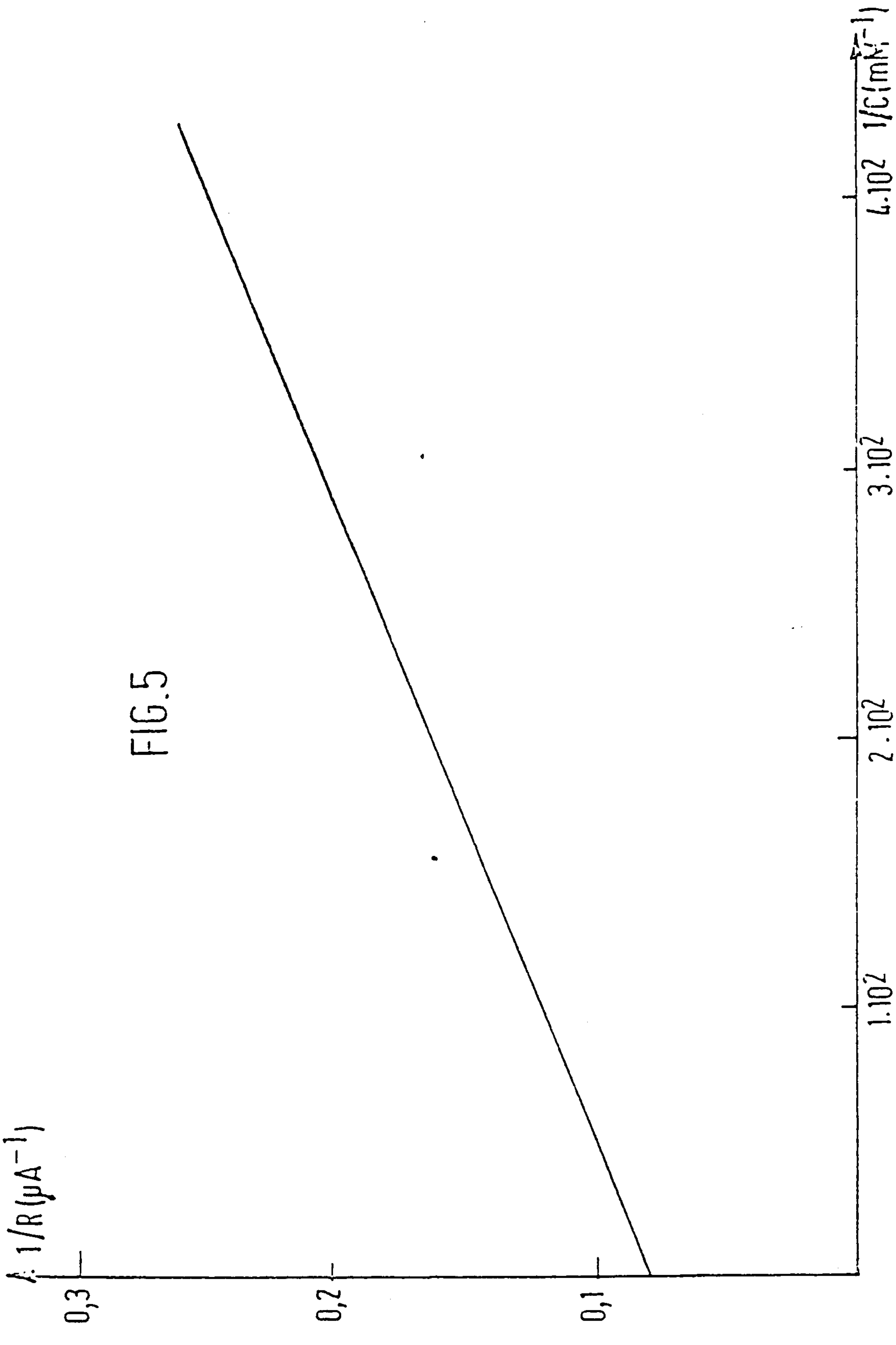


FIG.5

FIG.6

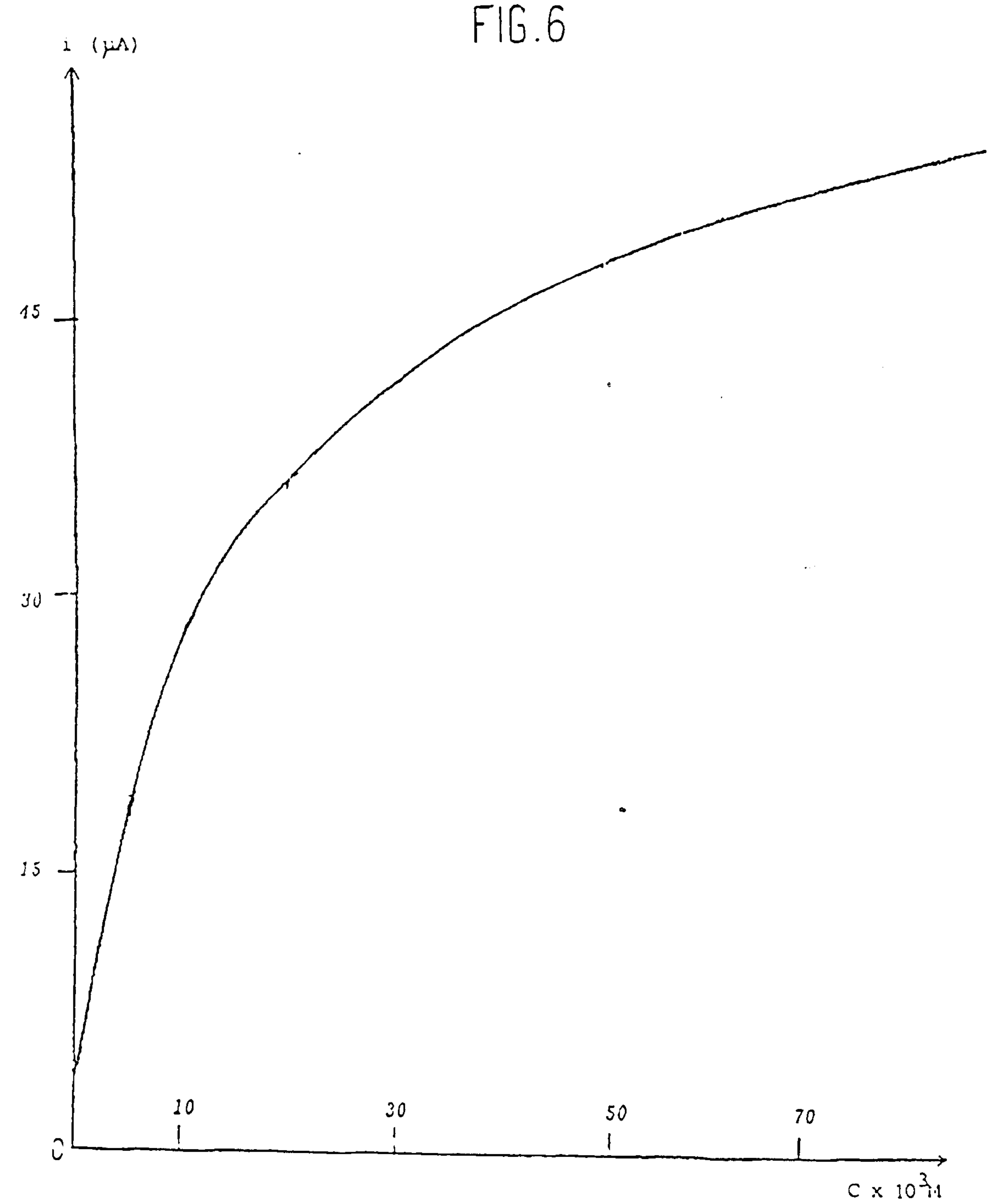

FIG. 7

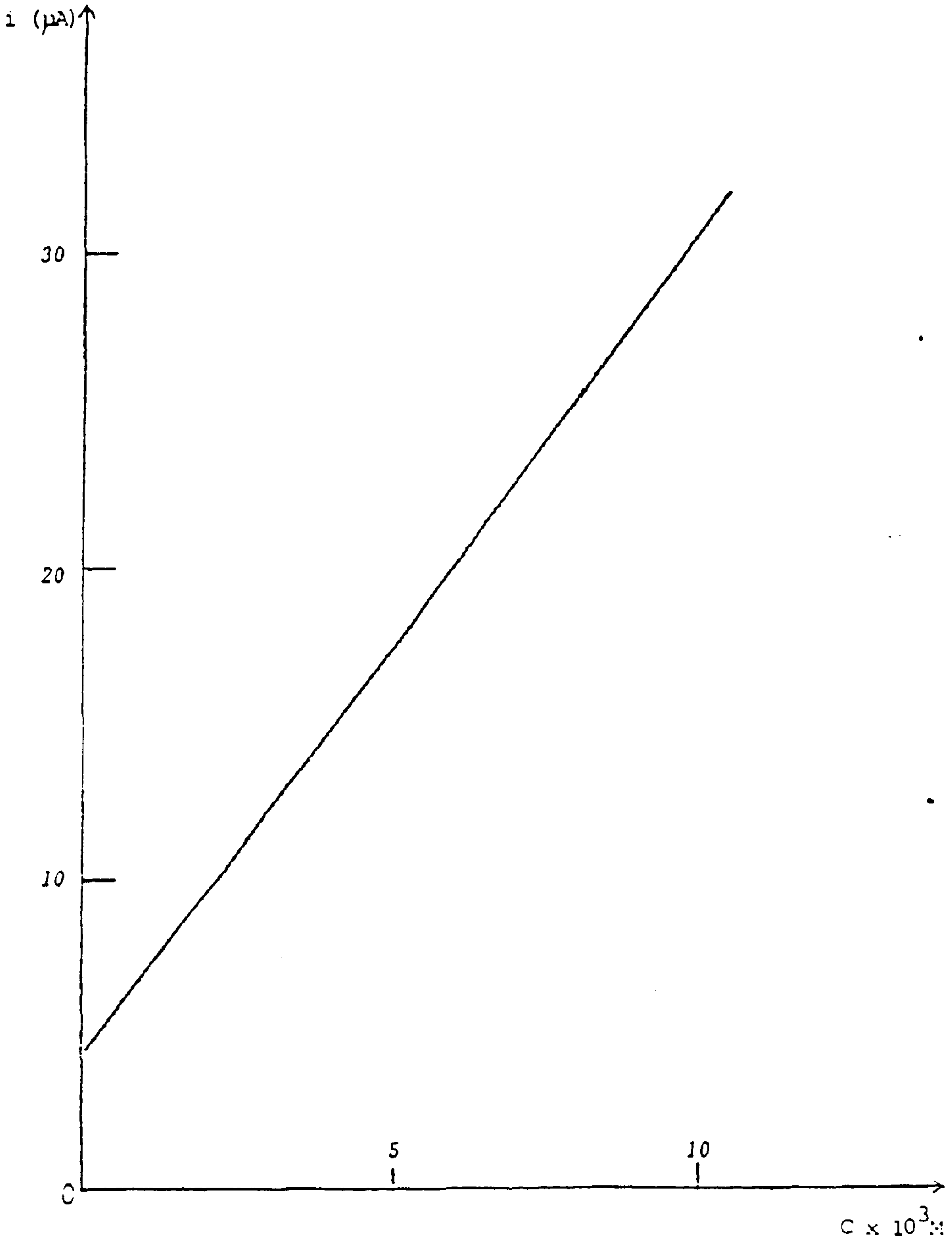

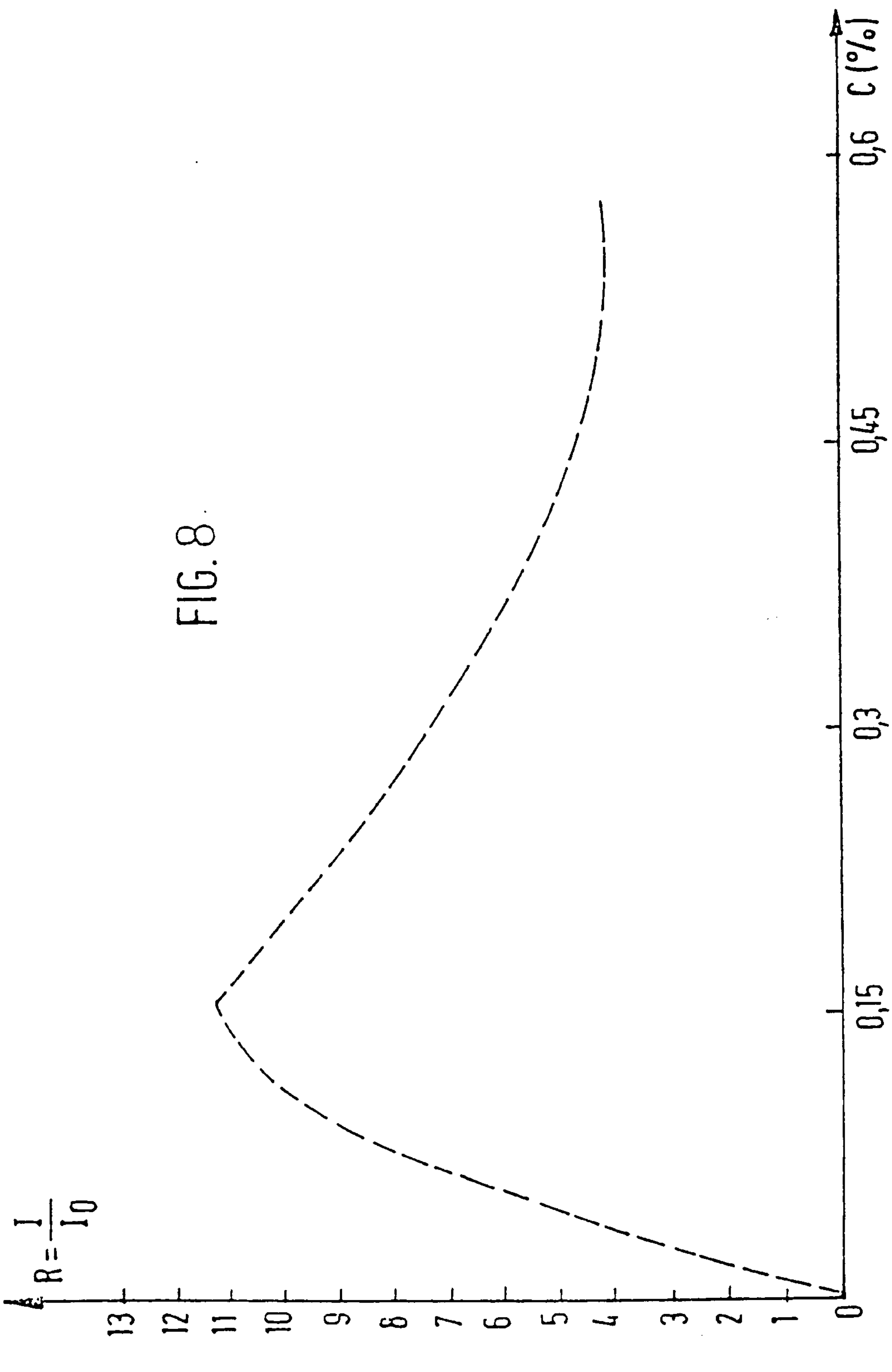


FIG. 8

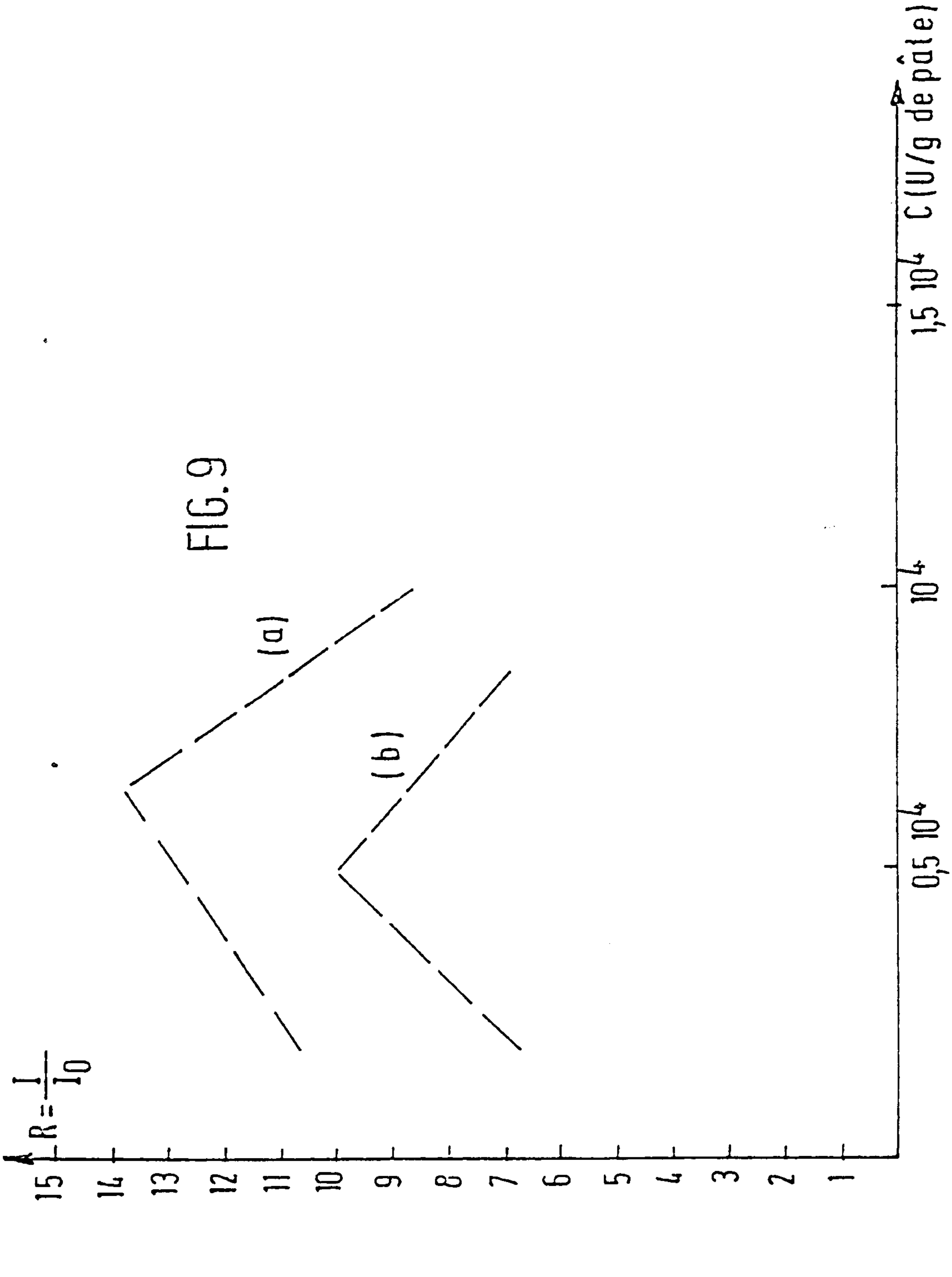


FIG. 9

FIG. 10

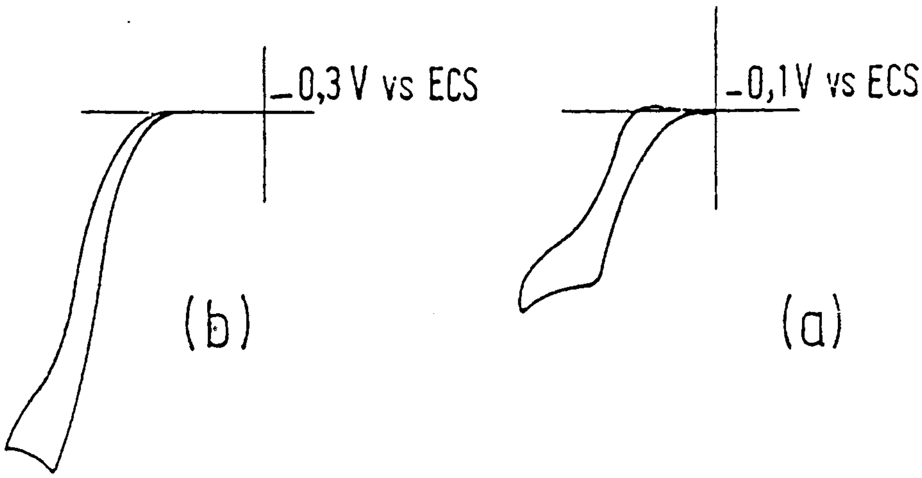


FIG. 11

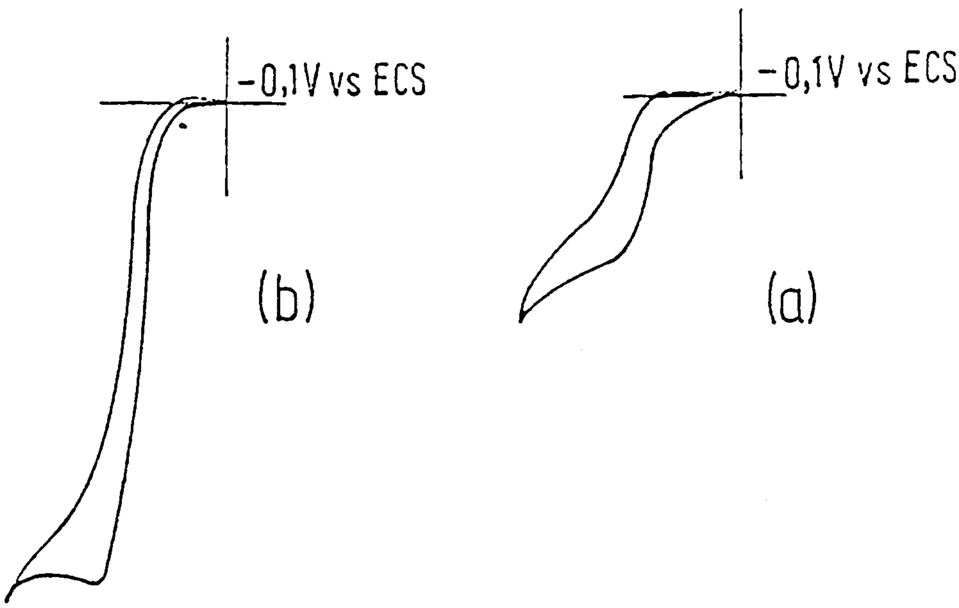

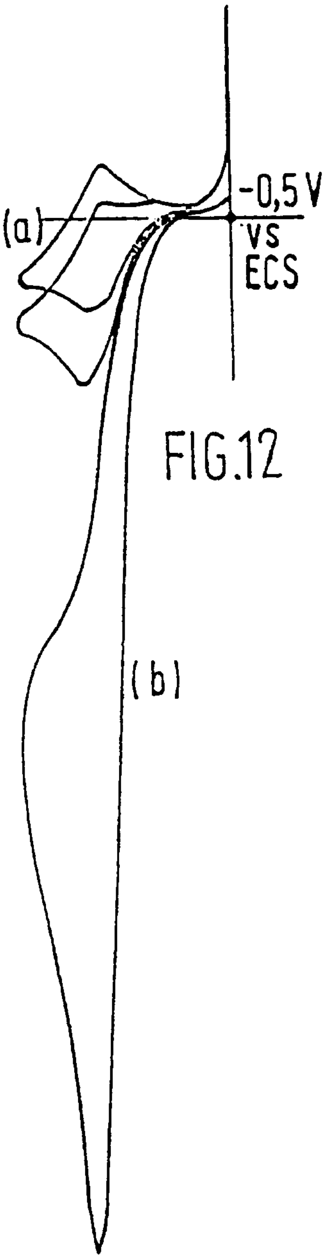


FIG.12

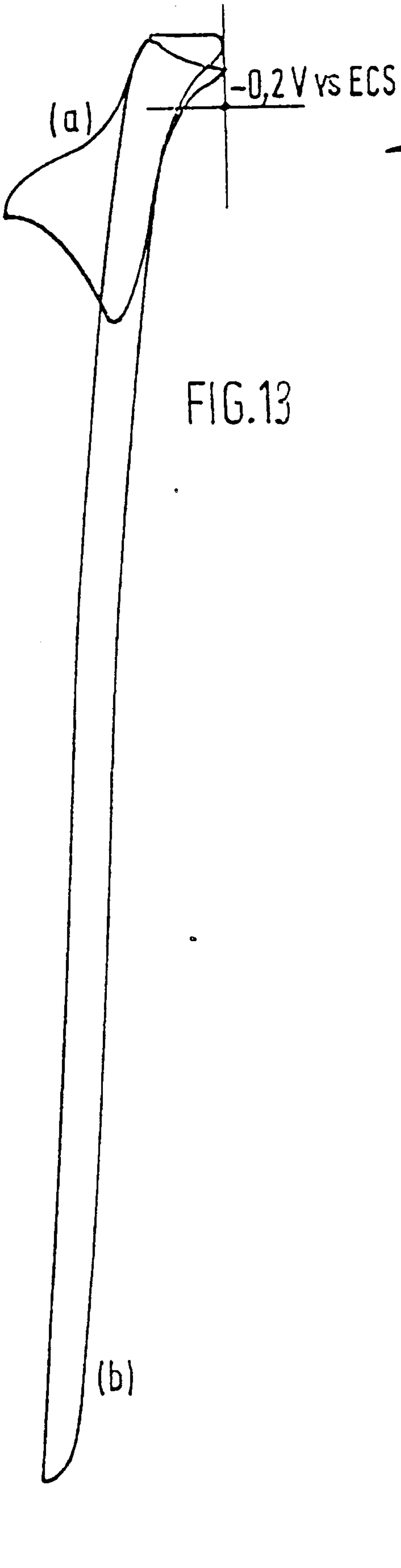


FIG.13

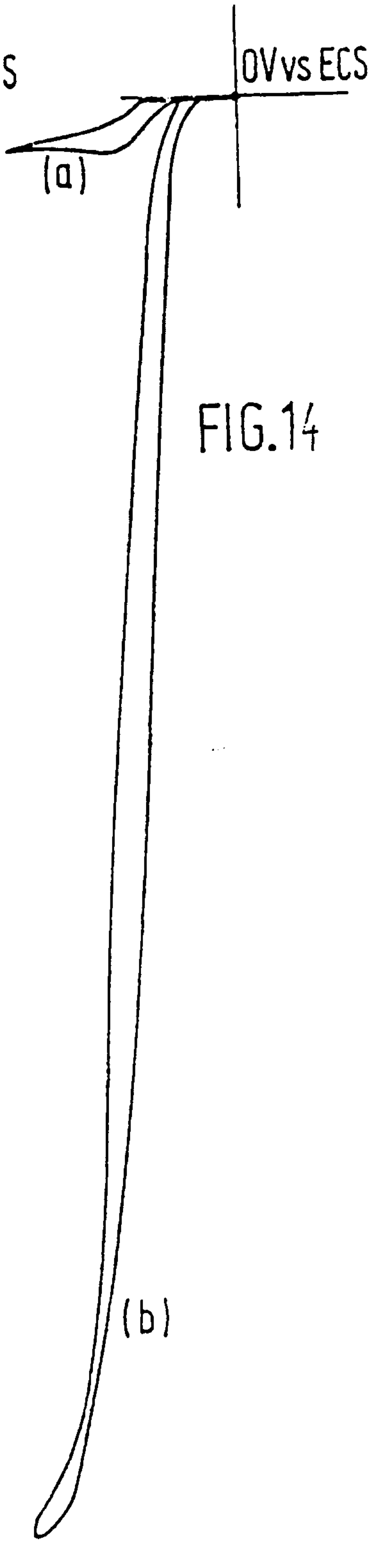


FIG.14

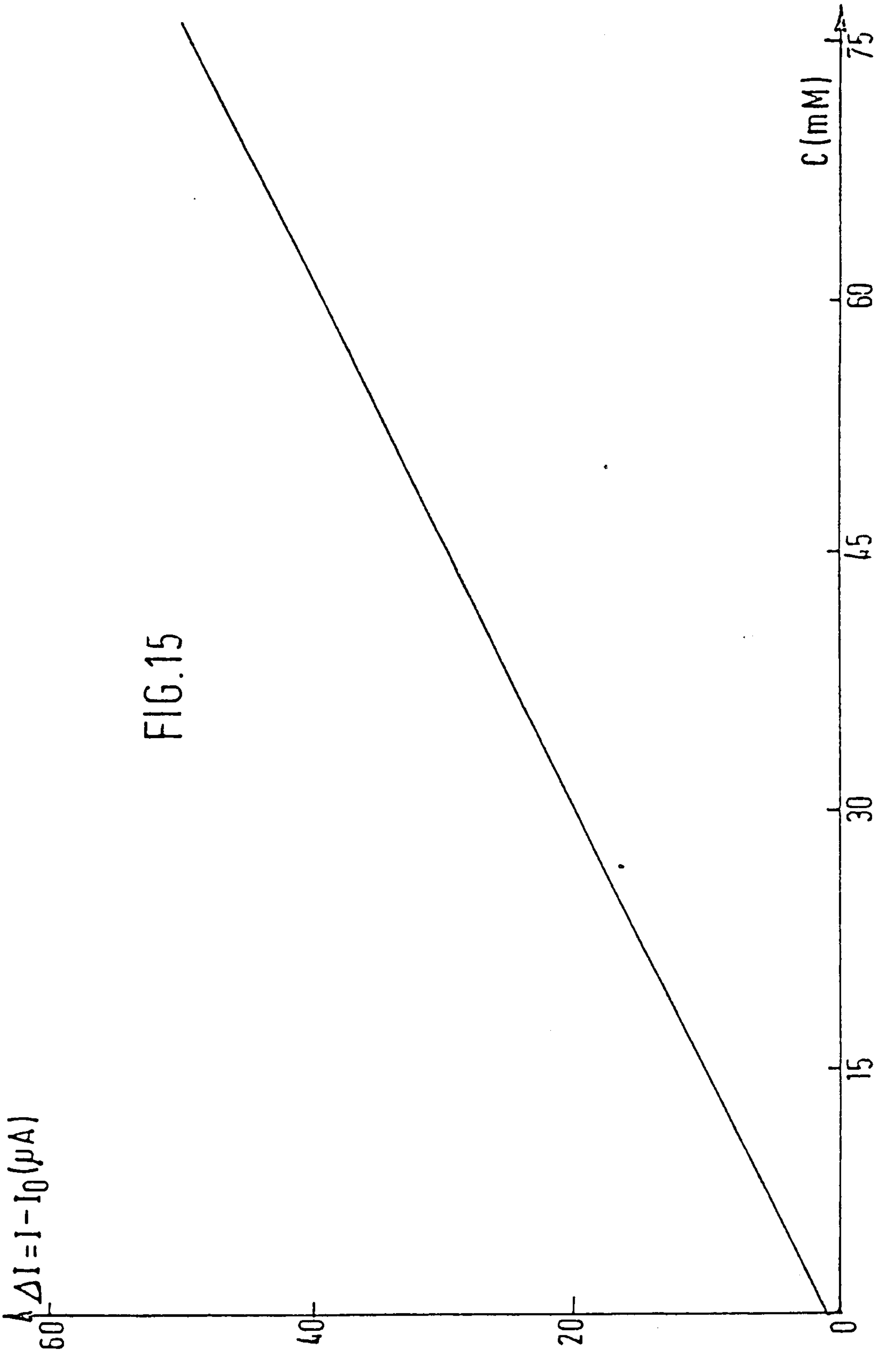


FIG.15

FIG.16

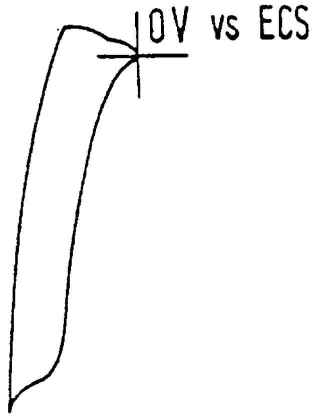


(a)

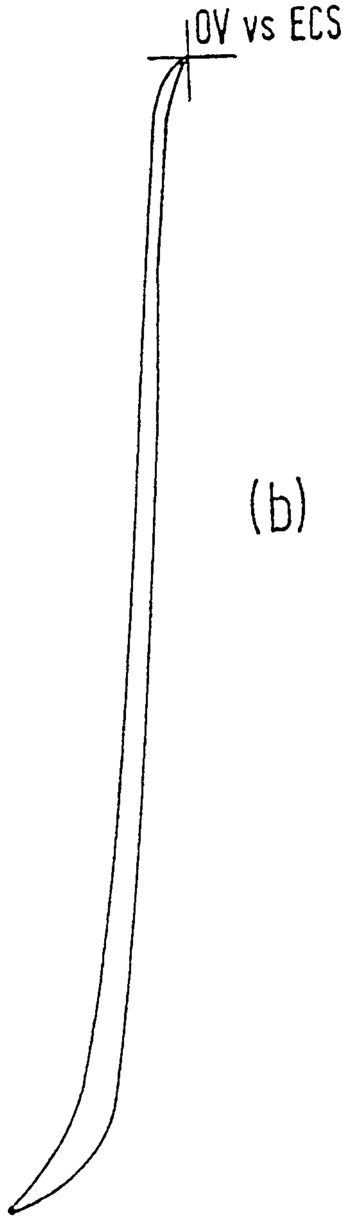


(b)

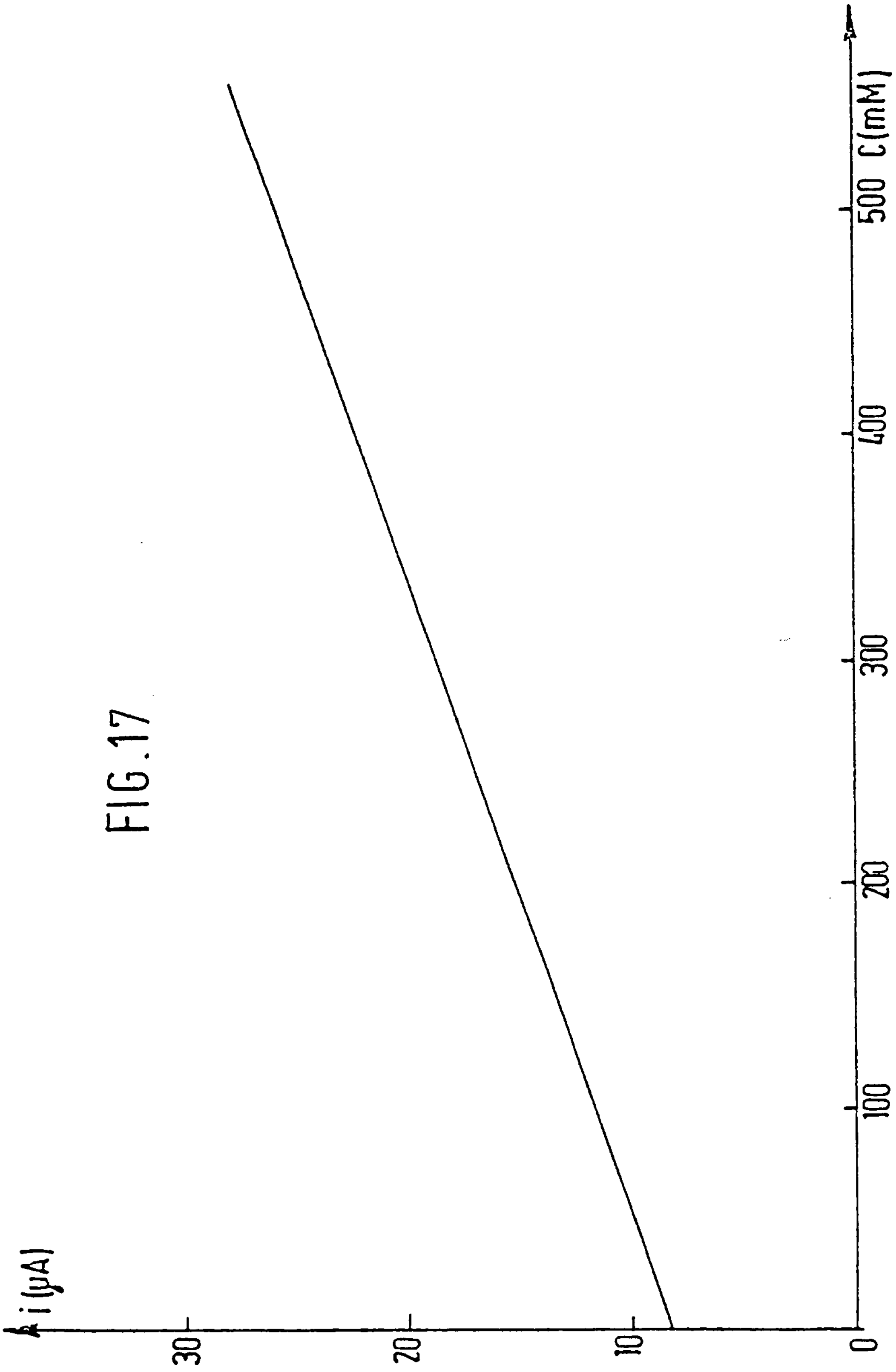


FIG.17

FIG.18

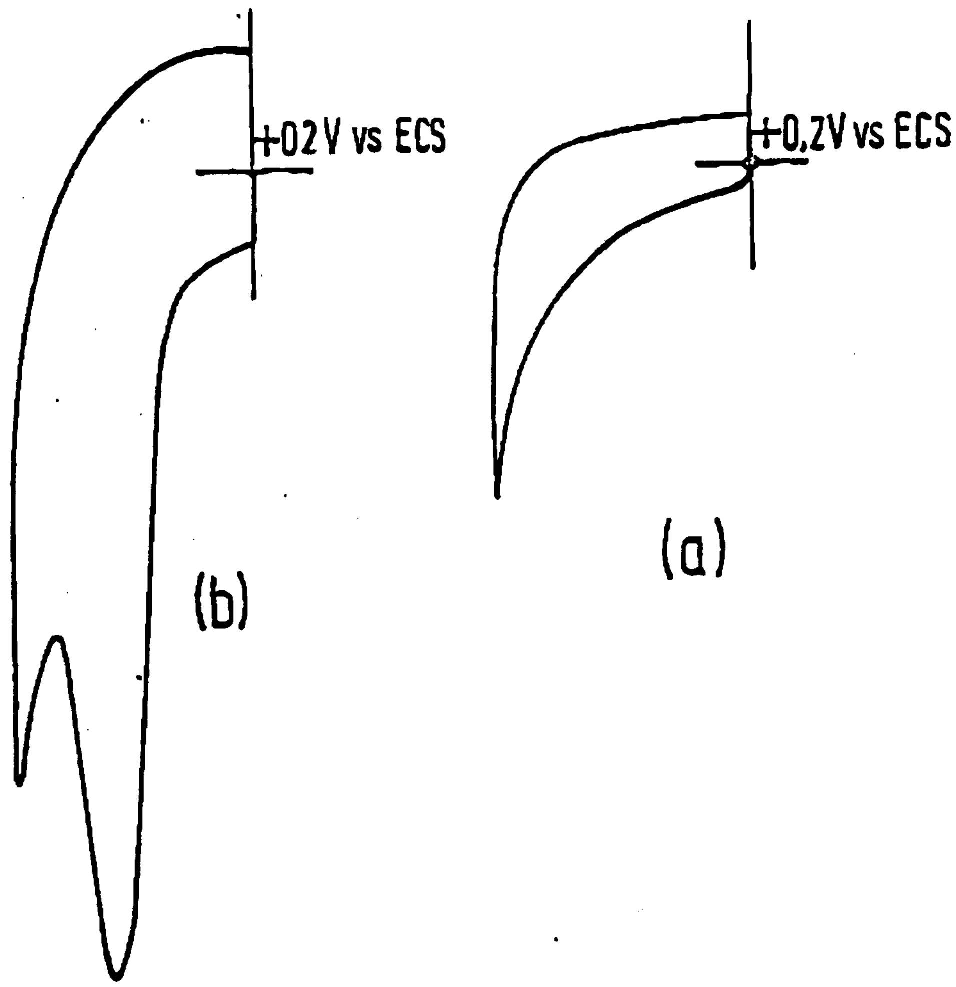

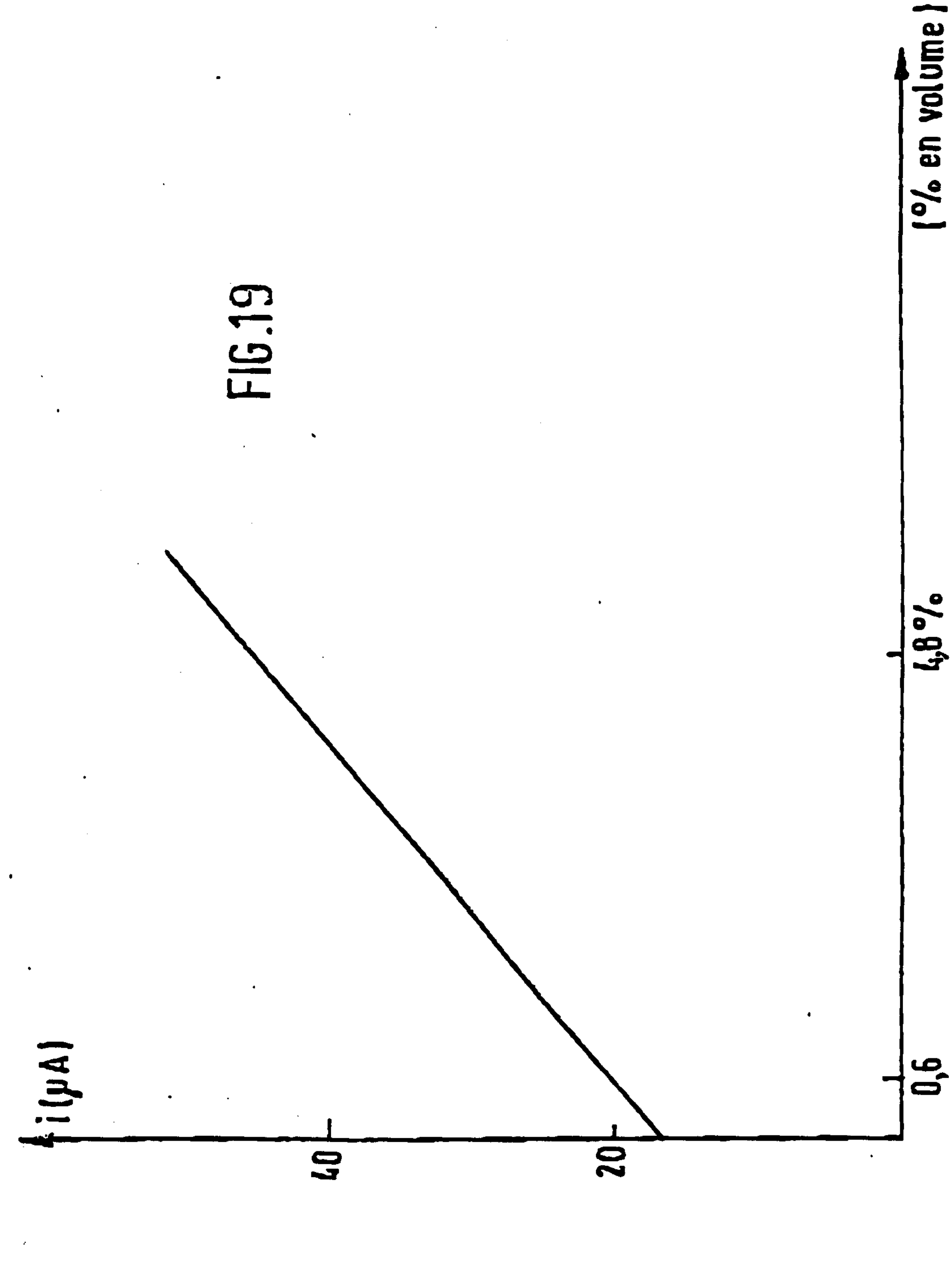


FIG.19

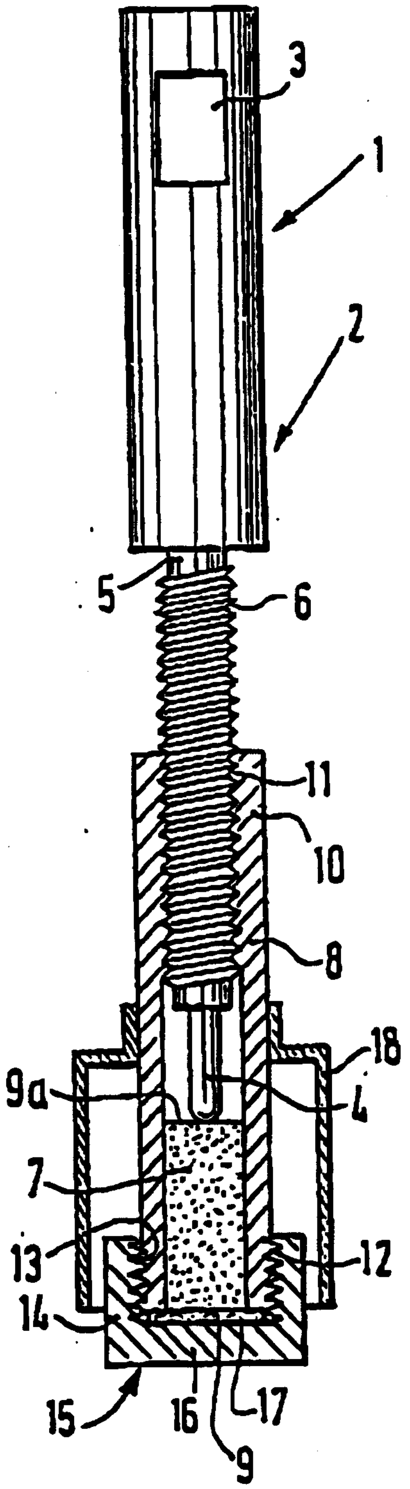


FIG.20